# EUROPEAN PATENT SPECIFICATION

(11) **EP 1 809 330 B1**
(45) Date of publication and mention of the grant of the patent: **27.04.2011**
(21) Application number: 05813305.9
(22) Date of filing: 14.10.2005
(51) Int. Cl.: A61K 33/24, A61K 47/28, A61K 31/575, A61K 47/36, A61P 35/00

(54) **COMPOSITIONS FOR REDUCING TOXICITY OF CISPLATIN, CARBOPLATIN, AND OXALIPLATIN**
ZUSAMMENSETZUNGEN ZUR VERRINGERUNG DER TOXIZITÄT VON CISPLATIN, CARBOPLATIN UND OXALIPLATIN
METHODES ET COMPOSITIONS POUR REDUIRE LA TOXICITE DU CISPLATINE, CARBOPLATINE, ET DE L'OXALIPLATINE

(30) Priority: 15.10.2004 US 619199 P
(43) Date of publication of application: 25.07.2007
(73) Proprietor: Yoo, Seo Hong, Wyckoff, New Jersey 07481 (US)
(72) Inventor: Yoo, Seo Hong, Wyckoff, New Jersey 07481 (US)
(74) Representative: Lucas, Phillip Brian
(86) International application number: PCT/US2005/037211
(87) International publication number: WO 2006/044771

(56) References cited:
- WO-A-2006/057637
- WO-A2-00/04875
- WO-A2-01/56547
- WO-A2-2006/026555
- WO-A2-2006/050165
- US-A1- 2001 046 521
- CANNON J B ET AL: "Reduction of pain on intravenous infusion with bile salt formulations for a macrolide antibiotic" INTERNATIONAL JOURNAL OF PHARMACEUTICS 1995 NETHERLANDS, vol. 114, no. 1, 1995, pages 65-74, XP002415758 ISSN: 0378-5173
- VILLANUEVA G R ET AL: "Effect of bile acids on hepatobiliary transport of cisplatin by perfused rat liver" PHARMACOLOGY AND TOXICOLOGY, vol. 80, no. 3, 1997, pages 111-117, XP008073844 ISSN: 0901-9928
- DOMINGUEZ MARIA F ET AL: "Low in vivo toxicity of a novel cisplatin-ursodeoxycholic derivative (Bamet-UD2) with enhanced cytostatic activity versus liver tumors" JOURNAL OF PHARMACOLOGY AND EXPERIMENTAL THERAPEUTICS, vol. 297, no. 3, June 2001 (2001-06), pages 1106-1112, XP002415759 ISSN: 0022-3565
- Kathleen Parfitt: "Martindale: The complete drug reference, 32nd edition" 1 January 1999 (1999-01-01), Pharmaceutical Press , London, UK , pages 629-630
- Kathleen Parfitt: "Martindale: The complete drug reference, 32nd edition" 1 January 1999 (1999-01-01), Pharmaceutical Press , London, UK , pages 607-609
- Kathleen Parfitt: "Martindale: The complete drug reference, 32nd edition" 1 January 1999 (1999-01-01), Pharmaceutical Press , London, UK , pages 556-558
- Kathleen Parfitt: "Martindale: The complete drug reference, 32nd edition" 1 January 1999 (1999-01-01), Pharmaceutical Press , London, UK , pages 513-515
- Kathleen Parfitt: "Martindale: The complete drug reference, 32nd edition" 1 January 1999 (1999-01-01), Pharmaceutical Press , London, Uk , page 593
- MITSUYOSHI H. ET AL: 'Ursodeoxycholic acid protects hepatocytes against oxidative injury via induction of antioxidants' BIOCHEMICAL AND BIOPHYSICAL RESEARCH COMMUNICATIONS 19990924 US vol. 263, no. 2, 24 September 1999, pages 537 - 542 ISSN: 0006-291X
- KEENE C. DIRK ET AL: 'A bile acid protects against motor and cognitive deficits and reduces striatal degeneration in the 3-nitropropionic acid model of Huntington's disease' EXPERIMENTAL NEUROLOGY, ACADEMIC PRESS, NEW YORK, NY, US vol. 171, no. 2, 01 October 2001, pages 351 - 360, XP002375047 ISSN: 0014-4886
- RODRIGUES C.M.P. ET AL: 'NEUROPROTECTION BY A BILE ACID IN AN ACUTE STROKE MODEL IN THE RAT' JOURNAL OF CEREBRAL BLOOD FLOW AND METABOLISM, RAVEN PRESS, LTD., NEW YORK, NY, US vol. 22, no. 4, 01 April 2002, pages 463 - 471, XP009064738 ISSN: 0271-678X
- LAPENNA D. ET AL: 'Antioxidant properties of ursodeoxycholic acid' BIOCHEMICAL PHARMACOLOGY 20021201 US vol. 64, no. 11, 01 December 2002, pages 1661 - 1667 ISSN: 0006-2952
- SUN AH PARK ET AL: "Cisplatin-induced apoptotic cell death in mouse hybrid neurons is blocked by antioxidants through suppression of cisplatin-mediated accumulation of p53 but not of Fas/Fas ligand" JOURNAL OF NEUROCHEMISTRY 2000 US, vol. 75, no. 3, 2000, pages 946-953, ISSN: 0022-3042

## Description

The present disclosure is related to clear aqueous solutions of one or more bile acids that may be used to ameliorate the toxic effects of a substance (e.g., a chemotherapeutic agent).

The full therapeutic value of some pharmaceutical compounds (e.g., medications, drugs) may be difficult or impossible to realize due to off-setting toxic effects. In some cases, one or more toxic effects of a pharmaceutical may be so significant, that the agent cannot be used safely in humans. In other cases, dosages may have to be limited to avoid a toxic effect. In still other cases, the course of therapy may have to be shortened. In addition, exigent circumstances or life-threatening illnesses may compel patients to simply endure such toxic effects to gain a pharmaceutical compound's benefits.

WO 01/56547 describes compositions for pharmaceutical and other uses comprising clear aqueous solutions of bile acids, an aqueous soluble starch conversion product and a pharmaceutical compound that may be lamivudin, stavudine, zalcitabine, d-penicillamine, amiodarone, lovastatin, pravastatin, simvastatin, metronidazole, colchicine and didanosine.

WO 00/04875 also describes compositions for pharmaceutical and other uses comprising clear aqueous solutions of bile acids, an aqueous soluble starch conversion product and a pharmaceutical compound that may be paclitaxel.

Villanueva, M.E. et al in Pharmacology & Toxicology, 1997, 80, 111-117 described the effect of solutions of bile acids on hepatobiliary transport of cisplatin by pefused rat iver.

Dominguez, M.F. et al in J. Pharmacol & Exp. Therapeutics, 2001, 297(3), 1106-1112 disclosed that the complexes Bamet-UD2 and Bamet-R2, both of which contain cisplatin and bile acid derivatives, exhibit less toxic effects than cisplatin, in vivo when administered to rats as a chemotherapeutic agent for treating liver tumours.

WO 2006/050165 describes clear aqueous solutions of one or more bile acids and an aqueous soluble starch conversion product which may be administered to a subject in conjunction with a pharmaceutical compound having a therapeutic effect on subjects with a neurogenerative disease and/or motor neuron disease.

Mitsuyoshi, H. et al in Biochemical & Biophysical Research Communications, 1999, 263 (2), 537-542 studied the effect of ursodeoxycholic acid on oxidative injury and antioxidative systems in cultured rat hepatocytes.

Keene, C.D. et al in Experimental Neurology, 2001, 171(2), 352 - 360 reported that tauroursodeoxycholic acid (TUDCA) significantly reduced 3-nitropropionic acid (3-NP) mediated striatal neuronal cell death in cell culture. In addition, rats treated with TUDCA exhibited an 80% reduction in apoptosis and in lesion volumes associated with 3-NP administration.

Rodrigues, C.M.P. et al in J. Cebral Blood Flow & Metabiolism, 2002, 22(4), 463-471 examined whether TUDCA could reduce the injury associated with acute stroke in a well-characterized model of transient focal cerebral ischemia. Their findings suggested that TUDCA could play a part in in vivo neuroprotection and thus be useful in the treatment of stroke.

Sun Ah Park et al in J. Neurochemistry, 2000, 75(3), 946-953 described a study in which they found that cisplatin-induced apoptic cell death in mouse hybrid neurons is blocked by antioxidants through suppression of cisplatin-mediated accumulation of p53 but not of Fas/Fas Ligand.

Lapenna, D. et al in Biochemical Pharmacology, 2002, 64(11), 1661-1667, investigated the potential antioxidant properties of ursodeoxycholic acid and found that it has direct antioxidant properties which are especially relevant against Fe³⁺ - and OH˙- dependent biomolecular oxidative damage.

Qiao, D. et al. in Carcinogenesis, 2001, 22(6), 957-964 investigated the effect of bile acids on the tumour suppressor p53 using the human colon tumour cell line HCT116. Their results suggested a novel signalling mechanism of bile acids that may play an important role in colon tumour promotion mediated by bile acids.

Therefore, it is an object of the invention to provide compositions that ameliorate or eliminate the toxicity of a pharmaceutical compound selected from cisplatin, carboplatin and oxaliplatin. This object can be achieved by the composition as defined in the independent claims. Further enhancements are characterized in the dependent claims.

The present disclosure relates to compositions that may ameliorate or eliminate a toxicity (e.g., a toxic effect) of a pharmaceutical compound. A method of ameliorating or eliminating a toxic effect of a pharmaceutical compound may include co-administering the pharmaceutical compound and a bile acid composition. The pharmaceutical compound used in the present invention is selected from the group consisting of cisplatin, carboplatin and oxaliplatin. The compositions to which the present invention is directed are defined in the claims.

Co-administration may include administration of one or more formulations at about the same time or during the same time period. For example, co-administration may include administering both a pharmaceutical compound and a bile acid in a single composition. It may also include simultaneous administration of a plurality of compositions. Alternatively, coadministration may include administration of a plurality of compositions at different times during the same period.

The present disclosure relates to compositions which comprise (1) a bile acid, a bile acid derivative, or a bile acid salt, (2) water, and (3) a sufficient quantity of maltodextrin such that the bile acid and the maltodextrin remain in solution at any pH within a selected pH range.

The disclosure further relates to a pharmaceutical composition which comprises (1) a bile acid, a bile acid derivative, or a bile acid salt, (2) water, (3) a pharmaceutical compound in a pharmaceutically appropriate amount, and (4) a sufficient quantity of maltodextrin such that the bile acid, the pharmaceutical compound, and the maltodextrin remain in solution at any pH level within a selected pH range. According to the disclosure, the pharmaceutical compound is selected from the group consisting of cisplatin, carboplatin and oxaliplatin.

Also disclosed are solution dosage forms of bile acid compositions. These dosage forms may have improved bioavailability and absorbability of a bile acid. When these compositions further contain a pharmaceutical compound, the may also have improved bioavailability and absorbability of the pharmaceutical compound.

Also disclosed is a composition which comprises (1) a bile acid, a bile acid derivative, or a bile acid salt, (2) water, and (3) a sufficient quantity of carbohydrate such that the bile acid component and the carbohydrate remain in solution at any pH within a selected pH range, wherein the carbohydrate is maltodextrin.

A combination therapy composition may be provided which may increase the intensity of a response to or efficacy of a pharmaceutical compound. Such a composition may permit administration of lower dosages of a pharmaceutical compound, attack a disease complex at different points, affect elimination and/or alter absorption of a pharmaceutical compound. Such a composition may lead to or contribute to a reduction in toxicity and/or side effects of a pharmaceutical.

A more complete and thorough understanding of the present embodiments and advantages thereof may be acquired by referring to the following description taken in conjunction with the accompanying drawings, wherein:
FIGURE 1A is a bar graph showing the results of a cell viability assay in which N18D3 hybrid neurons were incubated for 48 hours with increasing concentrations cisplatin (* p < 0.05; ** p < 0.001 compared with cisplatin treatment);
FIGURE 1B is a bar graph showing the results of a cell viability assay in which N18D3 hybrid neurons were incubated with 10 µM cisplatin for the indicated times;
FIGURE 2A is a micrograph showing N18D3 hybrid neurons incubated in a control solution;
FIGURE 2B is a micrograph showing N18D3 hybrid neurons incubated in a solution containing 10 µM cisplatin;
FIGURE 2C is a micrograph showing N18D3 hybrid neurons incubated in a solution containing 20 µM cisplatin;
FIGURE 3A is a fluorescence micrograph showing N18D3 hybrid neurons incubated in a control solution and stained with Hoechst 33258;
FIGURE 3B is a fluorescence micrograph showing N18D3 hybrid neurons incubated in a solution containing 10 µM cisplatin and stained with Hoechst 33258;
FIGURE 3C is a fluorescence micrograph showing N18D3 hybrid neurons incubated in a solution containing 20 µM cisplatin and stained with Hoechst 33258;
FIGURE 4 shows size-fractionated DNA from a commercial DNA ladder ("M"), N18D3 neurons incubated in a control solution ("CTL"), in a solution, containing 10 µM cisplatin ("Cis 10 µM"), and in a solution containing 20 µM cisplatin ("Cis 20 µM");
FIGURE 5A shows Western blots of N18D3 neurons incubated for 48 hours in a control solution ("0"), in a solution containing cisplatin at a concentration of 1 µM, 2, µM, 5 µM, 5 µM, 10 µM, or 20 µM using antibodies raised against p53, p21, BCL-2, BCL-XL, BAX, and β-actin;
FIGURE 5B shows Western blots' of N18D3 neurons incubated for the indicated times in 20 µM cisplatin using antibodies raised against p53, p21, BCL-2, BCL-XL, BAX, and β-actin;
FIGURE 6A is a bar graph showing the results of a cell viability assay in which N18D3 hybrid neurons were incubated with cisplatin and increasing concentrations of UDCA (* p < 0.05; ** p < 0.001);
FIGURE 6B is a bar graph showing the results of a cell viability assay in which N18D3 hybrid neurons were incubated with increasing concentrations of UDCA;
FIGURE 7A is a micrograph showing N18D3 hybrid neurons incubated in a control solution;
FIGURE 7B is a micrograph showing N18D3 hybrid neurons incubated in a solution containing 20 µM cisplatin;
FIGURE 7C is a micrograph showing N18D3 hybrid neurons incubated in a solution containing 20 µM cisplatin and 1 mg/mL UDCA;
FIGURE 8A is a fluorescence micrograph showing N18D3 hybrid neurons incubated in a control solution and stained with Hoechst 33258;
FIGURE 8B is a fluorescence micrograph showing N18D3 hybrid neurons incubated in a solution containing 20 µM cisplatin and stained with Hoechst 33258;
FIGURE 8C is a fluorescence micrograph showing N18D3 hybrid neurons incubated in a solution containing 20 µM cisplatin and 1 mg/mL UDCA and strained with Hoechst 33258;
FIGURE 9 shows size-fractionated DNA from a commercial DNA ladder ("M"), N18D3 neurons incubated in a control solution ("CTL"), in a solution containing 20 µM cisplatin ("Cis 20 µM"), and in a solution containing 20 µM cisplatin and 1 mg/mL UDCA ("Cis + UDCA");
FIGURE 10A shows Western blots of N18D3 neurons incubated in a control solution ("Vehicle"), in a solution containing cisplatin ("Cis (µM)"), in a solution containing UDCA ("UDCA (mg/mL) ") using antibodies raised against p53, p21, BCL-2, BCL-XL, BAX, and β-actin;
FIGURE 10B is a bar graph showing densitometric values of the p53 Western blot of FIGURE 10A;
FIGURE 10C is a bar graph showing densitometric values of the p21 Western blot of FIGURE 10A; and
FIGURE 11 shows Western blots of N18D3 neurons incubated in a control solution containing 20 µM cisplatin without pre-treatment ("CTL"), with pre-treatment in a solution lacking UDCA ("Ve") , or with pre-treatment in a solution having the indicated amount of UDCA.
FIGURE 12 is a bar graph showing the results of a cell viability assay in which A1 human hybrid neurons were incubated with 50 µM hydrogen peroxide and increasing concentrations of UDCA.

The therapeutic benefits of a pharmaceutical compound may be mitigated or even negated if that agent also displays toxicity. For example, although cisplatin and suramin are among the most effective chemotherapeutic agents, they are associated with sensory neuropathy in cancer patients following treatment. A target of cisplatin-induced or suramin-induced neurotoxicity is dorsal root ganglion (DRG) neurons. Animals chronically exposed to cisplatin or suramin exhibit disturbances in axonal transport and microtubule assembly of DRG neurons and apoptotic cell death in DRG neurons. Cisplatin generates oxygen radicals, which are one of the pathogenic intermediates following chemotherapy. Other agents, such as chemotherapeutics, statins, proteosome inhibitors, and antiretroviral agents may similarly target DRG neurons and/or may be associated with axonal degeneration.

While statins may cause peripheral neuropathy, the incidence may be low. In view of the beneficial effects of statins in terms of cardiovascular protection, this incidence may be regarded as relatively innocuous. The incidence of thalidomide neuropathy may be high (e.g., up to three quarters in some series). Although information on dose dependency is variable, lower cumulative doses may be less toxic. Like thalidomide, bortezomib, a novel proteosome inhibitor, is reportedly effective in the treatment of multiple myeloma and is associated with peripheral neuropathy. Oxaliplatin and epothilone are emerging anticancer drugs with neurotoxic potential. Similarly, leflunomide, a new disease modifying-agent approved for the treatment of rheumatoid arthritis, is reported to cause neuropathy. Antiretroviral agents may be associated with antiretroviral toxic neuropathies (ATN), disorders that may be characterized mostly by sensory symptoms that include "dying black" axonal degeneration of long axons in distal regions, loss of unmyelinated fibers, and variable degree of macrophage infiltration in peripheral nerves and dorsal root ganglia. Some antioxidants may effectively protect neurons from neurotoxicity although the mechanism of this neuroprotection has not been established. Cellular factors that may mediate toxicity and/or antioxidant neuroprotection include p53, Fas, and Fas ligand (Fas-L).

Ursodeoxycholic acid (3α-7β-dihydroxy-5β-cholanic acid) ("UDCA"), which may be a major component of bear bile, may be useful as a pharmaceutical compound for the treatment of and the protection against many types of disease (e.g., liver disease). Its medicinal uses may include the dissolution of radiolucent gall stones and treatment of biliary dyspepsia, primarily biliary cirrhosis, primary sclerosing cholangitis, chronic active hepatitis and hepatitis C.

In spite of the extremely valuable therapeutic activities and medical uses of bile acids as therapeutically active agents and as carriers and/or adjuvants, commercial use of bile acids has been limited to pharmaceutical formulations in which the bile acid is present in a solid form (*e.g*., tablets, capsules, and suspensions). This may be due to the insolubility of bile acids in aqueous media at pH from approximately 1 to 8. Bile has an extremely bitter taste and an equally bitter after-taste that lasts several hours both of which may be due to bile's insolubility. The few aqueous dosage forms that are available are unstable, and have very limited uses because of pH control and maintenance problems. Moreover, some commercial pharmaceutical dosage forms of bile acids have been shown to have scant bioavailability.

The present disclosure relates to clear, stable solutions of soluble bile acids that ameliorate or alleviate the toxicity of a pharmacological agent. Solutions of the disclosure may be used as delivery vehicles for a pharmacological agent with one or more toxic effects. Alternatively, solutions of the disclosure may be administered separately, in terms of both the route and time of administration. In some embodiments of the disclosure, a bile composition blocks a toxic effect mediated by p53. In some embodiments of the disclosure, a bile composition blocks a toxic effect mediated by an oxidative process.

A solution of the disclosure may be used to reduce or eliminate the neurotoxic effect of a pharmaceutical compound (*e.g*., a chemotherapeutic agent) in a human or non-human mammal. Solutions of the disclosure may also reduce or eliminate a toxic effect of an antineoplastic and/or an immunoactive drug (*e.g*., cisplatin, carboplatin, and oxaliplatin).

The present disclosure relates to an aqueous solution comprising (i) one or more soluble bile acids, aqueous soluble bile acid derivatives, or bile acid salts, (collectively "bile acid"), (ii) water, and (iii) maltodextrin in an amount sufficient to produce a solution which does not form a precipitate at any pH within a desired pH range. The composition may contain a bile acid or a bile acid salt which itself has pharmaceutical effectiveness. Formulations of the disclosure may act as a carrier, an adjuvant or enhancer for the delivery of a pharmaceutical material which remains dissolved in the composition of the disclosure across the desired pH range. Alternatively, according to some embodiments of the disclosure, the composition may comprise a non-bile acid pharmaceutical that is incompletely soluble.

In the present invention, the bile acid and the carbohydrate remain in solution without precipitation at any pH from acidic to alkaline. These aqueous solution systems of bile acid are substantially free of precipitate or particles. A further advantage of this disclosure is that the aqueous solution systems demonstrate no changes in physical appearance such as changes in clarity, color or odor following the addition of strong acids or alkali even after several months observation under accelerated conditions of storage at 50°C.

In some embodiments of the disclosure, an aqueous solution system of bile acid is administered orally whereupon it reaches the intestine through the gastrointestinal track without precipitation of bile acids by exposure to acidic gastric juices and alkaline juices of the intestine. These dissolved bile acid formulations demonstrate intact solution systems in the intestine can be effectively and completely absorbed and, consequently, undergo enterohepatic cycling. According to an embodiment of the disclosure, bile acid solubility (e.g. precipitation and changes in physical appearance) is affected by whether a carboxylic acid side chain of certain bile acids can be protonated (non-ionized), is ionized, or is a simple carboxylic acid.

The ionization state of a bile acid carboxylic acid side chain greatly effects the hydrophobicity and the hydrophillicity of the bile acid in these aqueous solution systems. The ionization state is manipulated by adjusting the pH to control the toxicity, absorption, and amphiphilicity of bile acids. One or more bile acids may be dissolved in these aqueous solution systems as a therapeutically active agent, as an adjuvant of a drug, as a carrier of a drug or as an enhancer of drug solubility. These aqueous solution systems may be prepared for oral consumption, enemas, mouthwashes, gargles, nasal preparations, otic preparations, injections, douches, topical skin preparations, other topical preparations, and cosmetic preparations which have a desired pH without the disadvantage of precipitation or deterioration in physical appearance after long periods of time.

Soluble bile acids are any type of aqueous soluble bile acids. A bile acid salt is any aqueous soluble salt of a bile acid. Bile salts exhibit greater solubilizing capacity for phospholipid and cholesterol and are consequently better detergents. More hydrophobic bile salts may be more injurious to various membranes, both in vivo and in vitro. Aqueous dissolved salts of bile acids may be formed by the reaction of bile acids described above and an amine including aliphatic free amines such as trientine, diethylene triamine, tetraethylene pentamine, and basic amino acids such as arginine, lysine, ornithine, and ammonia, and amino sugars such as D-glucamine, N-alkylglucamines, and quaternary ammonium derivatives such as choline, heterocyclic amines such as piperazine, N-alkylpiperazine, piperidine, N-alkylpiperidine, morpholine, N-alkylmorphline, pyrrolidine, triethanolamine, and trimethanolamine. According to the disclosure, aqueous soluble metal salts of bile acids, inclusion compound between the bile acid and cyclodextrin and its derivatives, and aqueous soluble O-sulfonated bile acids are also included as soluble bile acid salts.

Soluble bile acid derivatives, may be those derivatives which are in aqueous solution as soluble as or more soluble than is the corresponding underivatized bile acid. Bile acid derivatives include derivatives formed at the hydroxyl and carboxylic acid groups of the bile acid with other functional groups including halogens and amino groups. Soluble bile acid may include an aqueous preparation of a free acid form of bile acid combined with one of HCl, phosphoric acid, citric acid, acetic acid, ammonia, or arginine.

Bile acids that are used in accordance with the present invention are ursodeoxycholic acid, or a sodium salt of ursodeoxycholic acid.

In some embodiments of the instant disclosure, a major advantage may be that delivery of bile acid in solution achieves higher in vivo levels of bile acids than conventional preparations. Therefore, the therapeutic potential of bile acid may be more fully achieved than previous formulations. The in vivo levels of bile acids attainable with existing formulations in which bile is incompletely solubilized are lower and require administration of larger amounts of bile acids. Since bile acid is completely dissolved in the inventive formulations, higher in vivo levels of bile acid may be achieved, even though lower doses are administered.

Mixtures of two or more bile salts of differing hydrophobic activity may behave as a single bile salt of an intermediate hydrophobic activity. As a result, detergent properties and the toxicity of mixtures of two bile acids of differing hydrophobic activity often are intermediate between the individual components. The carbohydrate used in the present invention is the aqueous soluble starch conversion product maltodextrin obtained directly from the partial or incomplete hydrolysis of starch under various pH conditions. Examples include MALTRIN^{®} M700 a maltodextrin, manufactured by GPC^{®}, Grain Processing Corporation of Muscatine, Iowa.

The amount of high molecular weight aqueous soluble starch conversion product used in embodiments of the disclosure is at least the amount needed to render the chosen bile acid(s) in the preparation soluble in the concentration desired and in the pH range desired. According to the invention the minimal weight ratio of maltodextrin to UDCA required to prevent UDCA, precipitation is 6:1 (*i.e.* 1.2 g for every 0.2 g of UDCA, 6 g for every 1g of UDCA, and 12 g for every 2 g of UDCA in 100 mL of water).

In some embodiments of the disclosure, the formulation further comprises dietary fiber. Examples of dietary fiber include guar gum, pectin, psyllium, oat gum, soybean fiber, oat bran, corn bran, cellulose and wheat bran.

In some embodiments of the disclosure, the formulation further comprises emulsifying agents. For the purpose of the disclosure, the term "emulsifying agent" includes emulsifying agents and suspending agents. Examples of emulsifying agents include guar gum, pectin, acacia, carrageenan, carboxymethyl cellulose sodium, hydroxymethyl cellulose, hydroxypropyl cellulose, methyl cellulose, polyvinyl alcohol, povidone, tragacanth gum, xanthan gum, and sorbitan ester.

The selected pH range for which the formulation will not precipitate its bile acid, starch conversion product, or its pharmaceutical compound may be any range of pH levels obtainable with an aqueous system. This range is between about pH 1 and about pH 14 and more preferably between about pH 1 and about pH 10. Still more preferably the range is any subset of the range of pH levels obtainable in an aqueous system sufficient for a pharmaceutical formulation to remain in solution from preparation, to administration, to absorption in the body, according to the method of administration. Thus, the composition may be used as a pharmaceutical formulation wherein the pharmaceutical compound remains in solution without precipitation at prevailing pH levels in the mouth, stomach and intestines. In some embodiments of the disclosure, a bile acid remains dissolved under acidic conditions as a free bile acid in spite of the general insolubility of bile acids under acidic conditions.

In some embodiments of the invention, the pharmaceutical is cisplatin.

Some embodiments of the disclosure may be practiced with pH adjustable agents. Examples include HCl, H₃PO₄, H₂SO₄, HNO₃, CH₃COOH, citric acid, malic acid, tartaric acid, lactic acid, phosphate, eidetic acid and alkalies.

The formulations may be used to treat human and mammalian diseases. The disclosure contemplates treating gastrointestinal disorders, liver diseases, gall stones, and hyperlipidemia. Examples of liver diseases include alcohol-induced liver diseases and non-alcohol-induced liver diseases. Examples of gastrointestinal disorders include chronic gastritis, reflux gastritis, and peptic ulcer disease. Examples of non-alcohol-induced liver diseases include primary biliary cirrhosis, acute and chronic hepatitis, primary sclerosing cholangitis, chronic active hepatitis, and excess accumulation of fat in the liver. The disclosure further contemplates treating viral, bacterial and fungal diseases. A formulation may be administered to treat and/or eradicate *Helicobacter pylori* infection. A formulation may be administered to treat and/or eradicate hepatitis C virus infection, influenza A, Influenza C, parainfluenza 1, sendai, rubella, and pseudorabies virus. A formulation may be administered to treat acute or chronic inflammatory diseases. Examples of inflammatory diseases include bronchitis, chronic pharyngitis, and chronic tonsillitis.

A formulation may be administered to treat hypercholersterolemia.

The formulation may be modified such that it may be administered as a liquid, solid, powder or tablet. The formulation may be comprised in a solution, syrup, thick syrup or paste. An example of a solution is a solution of maltodextrin wherein the concentration of maltodextrin is less than 500 g/L. An example of a syrup is a solution of maltodextrin wherein the concentration of maltodextrin is between 500 g/L and 1.0 kg/L inclusive. An example of a thick syrup is a solution of maltodextrin wherein the concentration of maltodextrin is between 1.0 kg/L and 1.2 kg/L inclusive. An example of a paste is a solution of maltodextrin wherein the concentration of maltodextrin is greater than 1.2 kg/L.

The stability of dosage formulations of the disclosure may be evaluated by measuring the concentration of the relevant bile acid over time in preparations comprising soluble bile acid, a high molecular weight aqueous soluble starch conversion product, and water at various pH and temperature levels. The retention time (high performance liquid chromatography) of each bile acid may be adjusted as needed to permit individual analysis each bile acid present in complex samples, i.e. a sample having a plurality of bile acids. Stability tests may also be performed by assessing the light-scattering properties of a test solution. In addition, established accelerated testing conditions may be used.

All stability tests performed on solutions of the disclosure were satisfactory in that the concentration of bile acid as measured by HPLC did not change appreciably over time at various pH levels. Particularly, all bile acid solution formulations tested showed excellent results in the stability tests with no precipitation and no physical appearance changes over the test period. Some formulations remain stable for over 2 years. The aqueous solution dosage forms according to this disclosure that were tested did not change either physically or chemically at various pH conditions under the accelerated conditions despite the addition of therapeutically and chemically active agents that are stable and soluble in hydrochloric acid solution. Therefore, these aqueous solution systems may be extremely valuable pharmaceutical dosage forms for the therapeutically active bile acids preparations, and/or the drug (pharmaceutical compound) delivery preparations in which bile acids play roles as the adjuvant of drug, the carrier of drug, or the enhancer of solubility of a drug by micelle formation at various pH conditions without the stability problems, including precipitation in acidic conditions.

### EXAMPLES

The present disclosure may be better understood from the following examples.

### Example 1: Preparation of Bile Acid Solutions

A stock solution of bile acid was prepared by first dissolving UDCA (60 g) in 500 mL NaOH (6.7 g) solution. Next, to the resulting clear solution, 375' 9 of maltodextrin was added, portion by portion with vigorous agitation. The pH was then adjusted to between 7.0 and 7.2 by the dropwise addition of HCl with high throughput sonication (750W, 20kHz). The volume was then adjusted to 1.0 L with injectable distilled water: Lastly, the resulting clear solution was filter sterilized using a 0.22 µ GP express plus membrane under aseptic conditions. Dilutions of this solution to the desired UDCA concentration were prepared according to standard pharmacy practice.

### Example 2: Characterization of N18D3 Hybrid Neuronal Cell Line

The stable N18D3 mouse hybrid neuron line was used as a model neuronal cell culture system to investigate the pathomechanisms involved in cisplatin-induced neuropathy. This cell line is a hybrid between a mouse DRG neuron isolated from four-week-old Balb/C mice and the mouse neuroblastoma cell line N18TG2. N18D3 cells were grown in a 6-cm dish with Dulbecco's modified Eagle's medium containing 5% fetal bovine serum and 5% horse serum and incubated at 37°C in a humidified atmosphere with 5% CO₂.

Clonal N18D3 cells exhibited neuron-like properties not displayed by the parental neuroblastoma. For example, N18D3 hybrid neurons express a specific immunoreactivity to high molecular weight neurofilament protein (NF-H), indicating that they carry neuron-specific phenotypes. N18D3 hybrid neurons also expressed microtubule-associated protein-2 and low and medium molecular weight neurofilaments as shown by immunocytochemistry and sodium channel activity as shown by whole-cell clamp experiments.

### Example 3:. MTT Assay

Cell viability was assessed by measurement of 3-(4,'5'-Dimethylthiazol-2-yl) -2,5 diphenyltetrazolium bromide (MTT) reduction, which is an indicator of the pyridine nucleotide redox state of the cells. N18D3 cells (1 × 10⁵) were seeded onto 96-well plates in 0.1 ml of medium and twenty-four hours later were exposed to cisplatin and/or UDCA solutions according to Example 1. Cells were then incubated for pre-selected periods of time in the presence of cisplatin, UDCA, or both.

Ten microliters of MTT solution [5 mg/ml in phosphate-buffered saline (PBS)] was added to each well, and then the plates were incubated for 3 h at 37°C. After elution of the dye with dimethyl sulfoxide (Sigma), absorbance at 570 nm was measured in a dual-beam microtiter plate reader with 630-nm reference.

### Example 4: Nuclear Staining with Hoechst 33258

N18D3 cells grown on coverslips were treated with cisplatin for pre-selected times, fixed with 4% paraformaldehyde (pH 7.4) for 10 min, washed with PBS three times, and then incubated with 10 µg/ml Hoechst 33258 in PBS for 10 min. Changes in nuclear morphology were examined under an Olympus confocal fluorescence microscope.

### Example 5: DNA Fragmentation Analysis

Total DNA was extracted from the N18D3 hybrid neurons exposed to cisplatin or to cisplatin plus a 0.1 mg/mL UDCA solution prepared according to Example 1. To analyze DNA fragmentation patterns, cells were lysed in 5 mM Tris-HCl, pH 7.4, containing 0.5% TritonX-100 and 20 mM EDTA. Lysates were centrifuged at 16,090 g for 15 min at 4° C. Supernatants were extracted with phenol choloroform-isoamyl alcohol (25:24:1), precipitated in ethanol containing 0.3 M sodium acetate, and resuspended in Tris-EDTA buffer. The soluble DNA samples were subjected to electrophoresis on a 1.2% agarose gel.

### Example 6: Cisplatin Induces Apoptotic Cell Death in N18D3 Hybrid Neurons

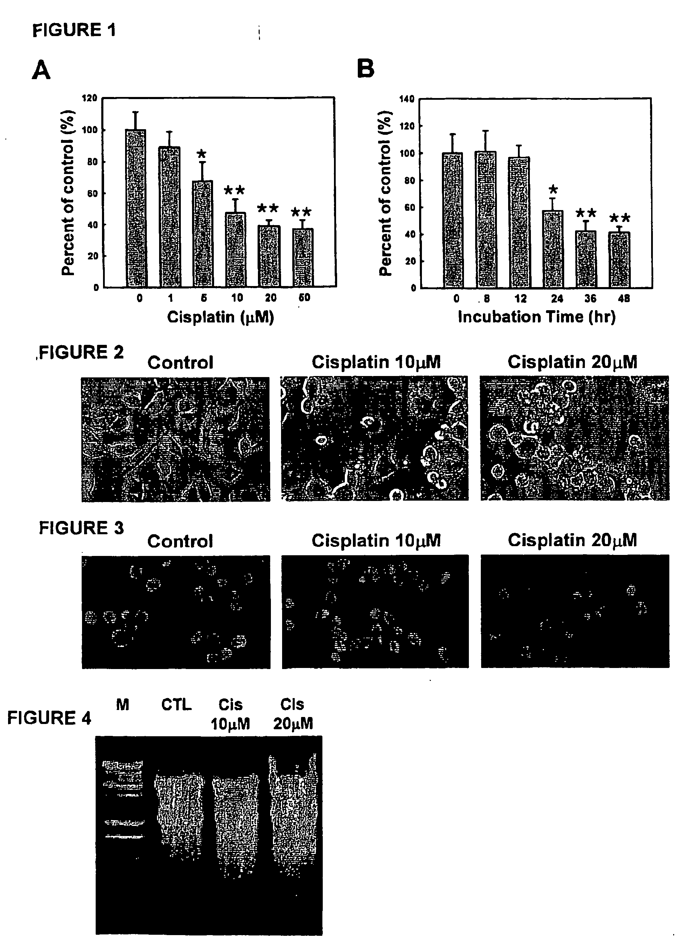

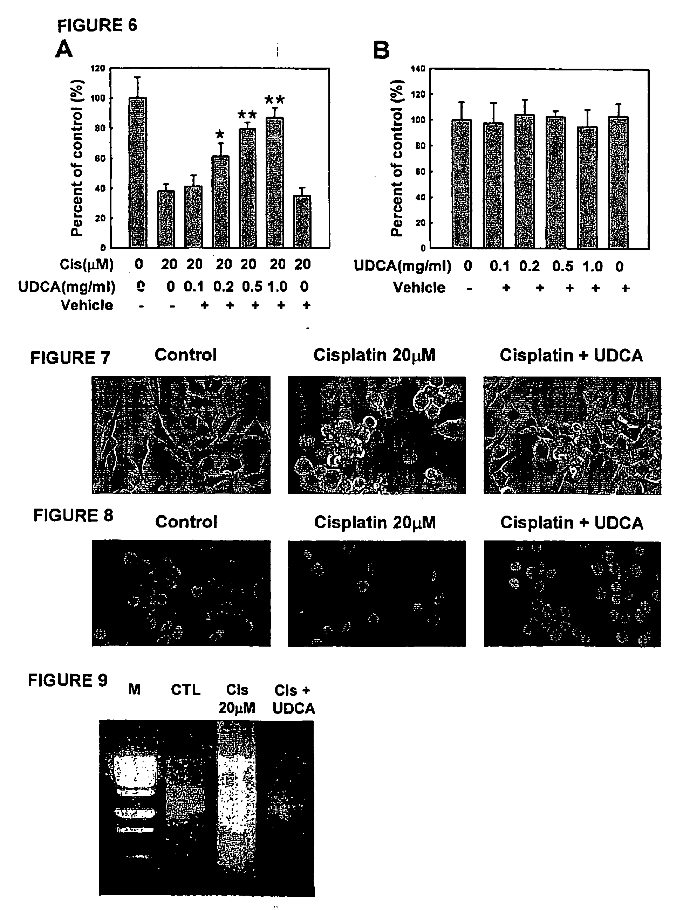

Cisplatin treatment of N18D3 neurons induced cytotoxicity in a dose- and time-dependent manner, as determined according to the MTT reduction assay of Example 3 (FIGURES 1A, 1B, 6A and 6B). Following exposure to 20 µM cisplatin for 36 h, cell viability decreased to 40% of untreated control cells (FIGURE 1A). Cisplatin induced cell death in N18D3 neurons with morphological changes such as cell shrinkage and cytoplasmic blebbing (FIGURES 2B, 2C, and 7B). N18D3 neurons exposed to cisplatin also showed condensed and fragmented nuclear changes, characteristic of apoptotic cell death, as shown by staining with Hoechst 33258 according to Example 4 (FIGURES 3B, 3C, and 8B). Moreover, cisplatin induced a clear 180- to 200-base internucleosomal DNA cleavage after cisplatin treatment according to the DNA fragmentation assay of Example 5 (FIGURES 4 and 9). These findings suggest that, without limiting the present disclosure to any particular mechanism of action, cisplatin-induced cytotoxicity in N18D3 hybrid neurons results from a cisplatin-mediated apoptotic pathomechanism.

### Example 7: UDCA Solution Substantially Reduced Cisplatin-Induced Apoptotic Cell Death in N18D3 Hybrid Neurons

N18D3 hybrid neurons were pretreated with a bile solution according to Example 1 for one hour prior to cisplatin treatment. Viability of neurons exposed to 20 µM cisplatin after being pretreated with a bile solution (1 mg/mL UDCA) was significantly increased as assessed by the MTT assay of Example 3 (FIGURE 6A). Pretreatment with a control solution prepared according to Example 1 except lacking UDCA had no effect on viability in the presence of cisplatin (right-most bar, FIGURE. 6A). In addition, bile solutions according to Example 1 had no adverse effect on cell viability as measured by MTT assay (FIGURE 6B).

Moreover, N18D3 hybrid neurons pretreated with the bile solution displayed fewer cisplatin-induced morphological changes such as cell shrinkage and cytoplasmic blebbing (FIGURE 7C) and no condensed or fragmented nuclear morphology (FIGURE 8C). Bile-pre-treated N18D3 hybrid neurons exposed to cisplatin for 36 hours lacked the internucleosomal DNA fragmentation observed in cells exposed to cisplatin without bile pre-treatment.

In this Example, the optimal concentration of UDCA for alleviating cisplatin-induced neurotoxicity was 1 mg/mL (equivalent to 0.1 mM of UDCA).

### Example. 8: Western Blot Analysis

N18D3 hybrid neurons were washed with PBS and lysed in RIPA buffer (50 nM Tris-HCl, pH 8.0, 150 mM NaCl, 1% NP-40, 0.5% sodium ceoxycholate, 0.1% sodium docecy sulfate containing 1 mM phenylmethylsulfonyl fluoride, and 1 µg/mL pepstatin A) on ice for 15 minutes. Cell lysates were cleared by centrifugation at 18,890 g for 15 minutes. Forty micrograms of protein, as determined by the Bio-Rad protein assay, was electrophoresed through 12% acrylamid-sodium dodecyl sulfate denaturing gels. Gels were transferred to Immobilon (Millipore, Bedford, MA) and probed with antibodies as recommended by the suppliers. Detection of specific protein was performed using an enhanced chemiluminiscence system.

### Example 9: Bile Acid Solutions Block. Accumulation of p53 and p21

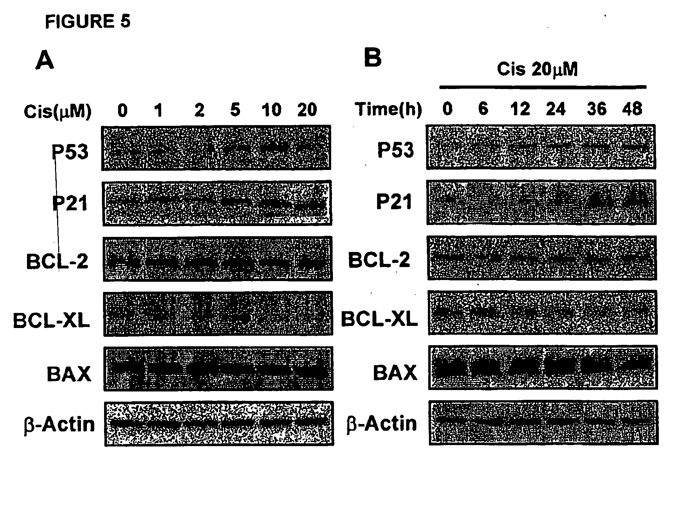

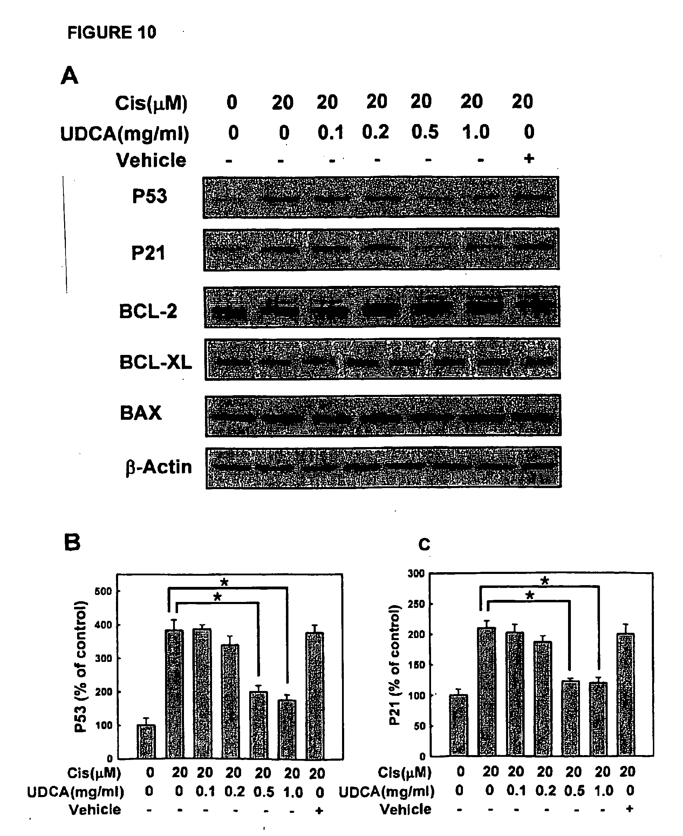

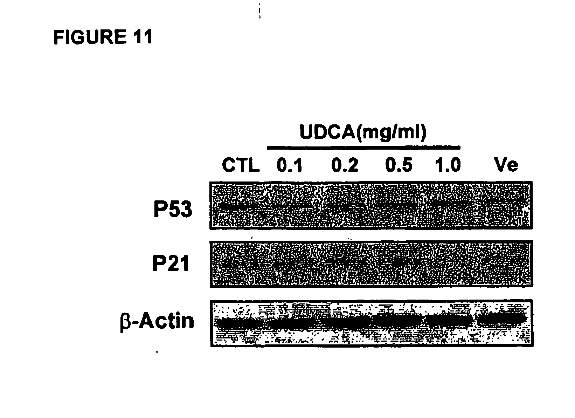

Key effector molecules and the regulatory mechanisms by which bile solutions exert neuroprotective effects in N18D3 hybrid neurons were investigated by assessing the expression of' various proteins previously implicated in apoptosis was determined. Accumulation of p53 protein increased four hours after treatment with 20 µM cisplatin, reached maximal level at twenty-four hours, and then slightly declined thereafter (FIGURE 5B). By contrast, bile-pre-treated neurons exhibit substantially less accumulation of p53 protein (FIGURES 10A, 10B, and 11).

These results are in accord with the results discussed in Examples 6 and 7, including the same optimal concentration of UDCA, namely 1 mg/mL. Without restricting the disclosure to any particular mechanism of action, these results suggest that cisplatin-induced accumulation of p53 is one of major clauses of apoptotic cell death of neurons and that suppression of p53 accumulation by bile solutions is a key requirement for neuroprotection.

In parallel with these p53 expression data, p21, a downstream target of activated p53, increased after four hours of cisplatin treatment, peaked at 8 hours, and declined thereafter, perhaps due to caspase activity. The bile acid solutions of Example 1 significantly suppressed accumulation of p21 (FIGURES 10A, 10C, and 11). This result is also in accord with he results of Examples 6 and 7, including the same optimal concentration of UDCA, namely 1 mg/mL.

In contrast, other downstream gene products of p53 including Bcl-2, Bcl-XL and Bax (Bcl2-associated X protein) showed no change in their expression following cisplatin treatment (FIGURE 10A). Again, without limiting the disclosure to any particular model, taken together, these results suggest that cisplatin-induced apoptosis is closely associated with p53 activation in neurons and that bile solutions of the disclosure may exert their neuroprotective effect through control of the p53 signaling pathway via p21.

### Example 10: Antioxidant Property of Bile Solutions of the Disclosure

Cell viability was assessed by measurement of MTT reduction, which is an indicator of the pyridine nucleotide redox state of the cells. A human hybrid neuronal cell line (1x10⁵)(Neurobiology of Disease 11, 184-198,2002) was seeded onto 96-well plates in 0.1 mL of medium and twenty-four hours later were exposed to the solution of Example 1. In addition, some cells were also exposed to hydrogen peroxide one hour after exposure to the bile solution (preincubation). Other cells were exposed to the bile solution at the same time as they were exposed to hydrogen peroxide. MTT assays were performed in accordance with Example 3.

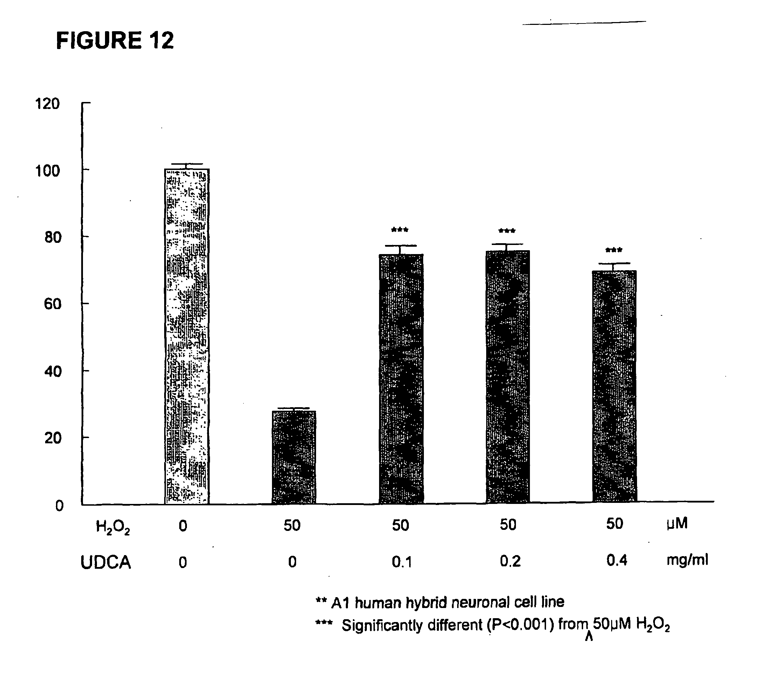

Results for pre-incubated cells, which were similar to simultaneously exposed cells, are shown in FIGURE 12. Relative to the untreated control, exposure to hydrogen peroxide alone resulted in the death of about 75% of the cells. However, almost 80% of the hybrid neurons pre-treated with a UDCA solution of the disclosure survived treatment with hydrogen peroxide. Without limiting the present disclosure to any particular mechanism of action, these data suggest that solutions of the disclosure have antioxidant properties.

### APPENDIX

**Table of Fig. 1A**

| *Treatment with Cisplatin for 48hr* |
|---|
| CTL: 100±11.2 (%, mean±SEM) |
| Cisplatin 1 µM: 89.0±9.7 |
| Cisplatin 5 µM: 67.3±12.3 |
| Cisplatin 10 µM: 47.2±8.4 |
| Cisplatin 20 µM: 39.0±3.6 |
| Cisplatin 50 µM: 37.1±5.7 |

**Table of Fig. 1B**

| *Treatment witch 20 µM cisplatin* |
|---|
| 0 hr: 100 ± 13.7 |
| 8 h: 101.2 ± 15.3 |
| 12 hr: 96.7 ± 8.9 |
| 24 hr: 57.6 ± 9.2 |
| 36 hr: 42.3 ± 7.6 |
| 48 hr: 41.3 ± 4.3 |

**Table 3 of Fig. 6A (n=6)**

| |
|---|
| Control = 100 ± 13.7 % |
| Cisplatin 20 µM = 38.2 ± 4.6 % |
| Cisplatin 20 µM + UDCA (0.1 mg/mL) = 41.3 ± 7.35 % |
| Cisplatin 20 µM + UDCA (0.2 mg/mL) = 61.3 ± 8.67 % |
| Cisplatin 20 µM + UDCA (0.5 mg/mL) = 79.2 ± 4.72 % |
| Cisplatin 20 µM + UDCA (1.0 mg/mL) = 86.7 ± 6.84 % |
| Cisplatin 20 µM + Vehicle = 35.2 ± 5.17 % |

**Table 3 of Fig. 6B (n=6)**

| |
|---|
| Control = 100 ± 13.7 % |
| UDCA (0.1 mg/mL) = 97.6 ± 15.7 % |
| UDCA (0.2 mg/mL) = 104.3 ± 11.6 % |
| UDCA (0.5 mg/mL) = 102.7 ± 4.8 % |
| UDCA (1.0 mg/mL) = 95.3 ± 13.2 % |
| Vehicle = 103.2 ± 9.7 % |

**Table 4 of Fig. 10B for P53 (n=3)**

| |
|---|
| Control = 100 ± 21.3 % |
| Cisplatin 20 µM = 383.5 ± 31.2 % |
| Cisplatin, 20 µM + UDCA (0.1 mg/mL) = 386.9 ± 13.5 % |
| Cisplatin 20 µM + UDCA (0.2 mg/mL) = 339.0 ± 27.3 % |
| Cisplatin 20 µM + UDCA (0.5 mg/mL) = 198.9 ± 19.7 % |
| Cisplatin 20 µM + UDCA (1.0 mg/mL) = 175.1 ± 15.2 % |
| Cisplatin 20 µM + Vehicle = 376.35 ± 22.6 % |

**Table 4 of Fig. 10C for P21**

| |
|---|
| Control = 100 ± 9.7 % |
| Cisplatin 20 µM = 209.7 ± 11.8 % |
| Cisplatin 20 µM + UDCA (0.1 mg/mL) = 202.2 ± 13.6 % |
| Cisplatin 20 µM + UDCA (0.2 mg/mL) = 186.7 ± 10.3 % |
| Cisplatin 20 µM + UDCA (0.5 mg/mL) = 122.3 ± 4.7 % |
| Cisplatin 20 µM + UDCA (1.0 mg/mL) = 119.8 ± 8.8 % |
| Cisplatin 20 µM + Vehicle = 200.15 ± 15.2 % |

| |
|---|
| * p < 0.05 ** P < 0.01 |

## Claims

1. A clear aqueous solution comprising:
(a) ursodeoxycholic acid or a sodium salt of ursodeoycholic acid (UDCA);
(b) maltodextrin;
(c) a pharmaceutically effective amount of a chemotherapeutic compound selected from the group consisting of cisplatin, carboplatin, and oxaliplatin; and
(d) water,
wherein the minimal weight ratio of maltodextrin to UDCA is 6:1 and the UDCA and maltodextrin both remain in solution at all pH values from about pH 1 to about pH 14.

2. The clear aqueous solution of claim 1, wherein the UDCA is present in a neuroprotective amount.

3. A kit for ameliorating or eliminating a toxic effect of a pharmaceutical compound selected from the group consisting of cisplatin, carboplatin and oxaliplatin comprising:
(I) a first composition comprising a pharmaceutical compound selected from the group consisting of cisplatin, carboplatin, and oxaliplatin; and
(II) a clear aqueous solution comprising:
(a) ursodeoxycholic acid or a sodium salt of ursodeoxycholic acid (UDCA);
(b) maltodextrin;
(c) water,
wherein the minimal weight ratio of maltodextrin to UDCA is 6:1 and the UDCA and the maltodextrin both remain in solution at all pH values from about pH 1 to about pH 14.

4. A kit according to claim 3, wherein the pharmaceutical compound is cisplatin.

## Patentansprüche

1. Klare, wässrige Lösung, die Folgendes umfasst:
(a) Ursodeoxycholsäure oder ein Natriumsalz von Ursodeoxycholsäure (UDCA);
(b) Maltodextrin;
(c) eine pharmazeutisch wirksame Menge einer chemotherapeutischen Verbindung, die aus der Gruppe bestehend aus Cisplatin, Carboplatin und Oxaliplatin ausgewählt ist; und
(d) Wasser,
wobei das minimale Gewichtsverhältnis von Maltodextrin zu UDCA 6:1 beträgt und sowohl UDCA als auch Maltodextrin beide bei allen pH-Werten im Bereich zwischen einem pH-Wert von ungefähr 1 bis zu einem pH-Wert von ungefähr 14 in Lösung bleiben.

2. Klare, wässrige Lösung nach Anspruch 1, wobei die UDCA in einer neuroprotektiven Menge vorhanden ist.

3. Kit zur Minderung oder Eliminierung einer toxischen Wirkung einer pharmazeutischen Verbindung , die aus der Gruppe bestehend aus Cisplatin, Carboplatin und Oxaliplatin ausgewählt ist und Folgendes umfasst:
(I) eine erste Zusammensetzung , die eine pharmazeutische Verbindung umfasst, die aus der Gruppe bestehend aus Cisplatin, Carboplatin und Oxaliplatin ausgewählt ist; und
(II) eine klare, wässrige Lösung, die Folgendes umfasst:
(a) Ursodeoxycholsäure oder ein Natriumsalz von Ursodeoxycholsäure (UDCA);
(b) Maltodextrin;
(c) Wasser,
wobei das minimale Gewichtsverhältnis von Maltodextrin zu UDCA 6:1 beträgt und sowohl UDCA als auch Maltodextrin bei allen pH-Werten im Bereich zwischen einem pH-Wert von ungefähr 1 bis zu einem pH-Wert von ungefähr 14 in Lösung bleiben.

4. Kit nach Anspruch 3, wobei die pharmazeutische Verbindung Cisplatin ist.

## Revendications

1. Solution aqueuse limpide comprenant :
(a) l'acide ursodésoxycholique ou un sel sodique de l'acide ursodésoxycholique (UDCA) ;
(b) la maltodextrine ;
(c) une quantité pharmaceutiquement efficace d'un composé chimiothérapeutique choisi dans le groupe consistant de cisplatine, carboplatine et oxaliplatine ; et
(d) l'eau,
dans laquelle le rapport de poids minimal de maltodextrine à l'UDCA est de 6:1 et l'UDCA et la maltodextrine restent tous les deux en solution à toutes les valeurs de pH d'environ 1 à environ pH 14.

2. Solution aqueuse limpide selon la revendication 1, dans laquelle l'UDCA est présent en une quantité neuroprotectrice.

3. Kit pour l'amélioration ou l'élimination d'un effet toxique d'un composé pharmaceutique sélectionné à partir du groupe composé de cisplatine, carboplatine et oxaliplatine comprenant :
(I) une première composition comprenant un composé pharmaceutique sélectionné à partir du groupe composé de cisplatine, carboplatine et oxaliplatine ; et
(II) une solution aqueuse limpide comprenant :
(a) l'acide ursodésoxycholique ou un sel sodique de l'acide ursodésoxycholique (UDCA) ;
(b) la maltodextrine ;
(c) l'eau,
dans laquelle le rapport de poids minimal de maltodextrine à l'UDCA est de 6:1 et l'UDCA et la maltodextrine restent tous les deux en solution à toutes les valeurs de pH d'environ 1 à environ pH 14.

4. Kit selon la revendication 3, dans lequel le composé pharmaceutique est la cisplatine.
